# EUROPEAN PATENT SPECIFICATION

(11) **EP 2 027 263 B1**
(45) Date of publication and mention of the grant of the patent: **11.07.2012**
(21) Application number: 07729324.9
(22) Date of filing: 21.05.2007
(51) Int. Cl.: C12N 9/94, A61K 35/12

(54) **PROCESS FOR SEPARATING AND DETERMINING THE VIRAL LOAD IN A PANCREATIN SAMPLE**
VERFAHREN ZUR TRENNUNG UND BESTIMMUNG DER VIRUSLAST IN EINER PANKREATINPROBE
PROCÉDÉ POUR SÉPARER ET DÉTERMINER LA CHARGE VIRALE DANS UN ÉCHANTILLON DE PANCRÉATINE

(30) Priority: 22.05.2006 EP 06114329
(43) Date of publication of application: 25.02.2009
(73) Proprietor: Abbott Products GmbH, 30173 Hannover (DE)
(72) Inventor: BECHER, Dietmar, 17498 Diedrichshagen (DE); DÖHNER, Leopold, 17489 Greifswald (DE); RÜFFER, Frauke c/o Solvay Pharmaceuticals GmbH, 30173 Hannover (DE); FRINK, Martin, 30900 Wedemark (DE)
(74) Representative: Bauriegel, Lutz
(86) International application number: PCT/EP2007/054880
(87) International publication number: WO 2007/135125

(56) References cited:
- EP-A- 1 593 688
- WO-A-91/16060
- WO-A-95/15681
- WO-A-98/38292
- DE-A1- 19 856 415

## Description

The present invention relates to a process for substantially quantitatively separating the viral load from a pancreatin specimen and to a process for quantitatively determining the viral load of this pancreatin specimen.

Pancreatin is a long-known mixture of various physiologically active constituents obtained from mammalian pancreatic glands. The main constituents of pancreatin are digestive enzymes, in particular pancreatic lipase, but also amylases and proteases. Thanks to its valuable therapeutic properties and high level of safety in use, pancreatin has long been used highly successfully as a pharmaceutical preparation in enzyme replacement therapy. Pancreatic lipase is of greatest significance here, but the amylases and proteases also make a considerable contribution to the therapeutic benefits of pancreatin. Pancreatin for therapeutic purposes is usually obtained from cattle ("bovine pancreatin") or pigs ("porcine pancreatin"), with porcine pancreatin being of the greatest significance in quantity terms. Methods for the production of pancreatin for therapeutic purposes are known per se, for example from the publication EP 0 115 023 A.

Due to the nature of animal derived pancreatin, the starting materials may typically be accompanied by unwanted biological components, such as bacterial or viral contaminants. However, during more than 100 years of commercialization of pharmaceutical products containing pancreatin, no case has been reported where patients have been affected by viral-contaminated pancreatin. Nevertheless, companies producing pharmaceutical products derived from biological tissues and/or body fluids experience increasing pressure from the regulatory bodies to increase the level of safety of their products by reducing all contaminants to the lowest level possible, independent of whether any concerned contaminant is considered a human pathogen or not. For the application of pancreatin in pharmacological products, it is therefore desirable to have reliable analytical methods for detecting and quantifying such biological contaminants.

To date, no reliable method has been developed for quantitatively detecting or separating viral contaminants in a pancreatin sample. This is likely due to the fact that the enzymatically active constituents of pancreatin are incompatible with the cell lines typically used for multiplying viruses using techniques known to a person of ordinary skill in the art, thus making it more difficult or even impossible to determine the virus titer in a pancreatin sample.

It was accordingly the object of the invention to provide a process for substantially quantitatively separating the viral load from a pancreatin specimen and a process for quantitatively determining the viral load of this pancreatin specimen. In particular, it was an object of the invention to provide a process for substantially quantitatively separating an infectious viral load from a pancreatin specimen and a process for quantitatively determining the infectious viral load of this pancreatin specimen.

It has now surprisingly been found that the viral load, in particular the infectious viral load, of a pancreatin specimen may be substantially quantitatively determined if the viral load is first separated from the pancreatin specimen in a multistage centrifugation process and the separated viral load is then quantitatively determined using per se known virological methods.

Publication JP 12856990 has already disclosed a method for concentrating or isolating hepatitis viruses by a nonspecific combination of low-speed centrifugation and ultracentrifugation.

In a first embodiment, the invention relates to a process for separating the viral load from a pancreatin specimen, comprising the process steps:
a) producing a liquid pancreatin test sample suitable for centrifugation from the pancreatin specimen without in so doing changing the viral load thereof,
b) subjecting at least one defined part of the pancreatin test sample from process step a) to at least one low-speed centrifugation under conditions, under which viruses with sedimentation constants of ≥ 120 S do not yet form a pellet,
c) discarding any solid deposit optionally arising in process step b) during low-speed centrifugation and retaining a pancreatin test sample supernatant,
d) subjecting at least one defined part of the pancreatin test sample supernatant obtained in process step c) to ultracentrifugation in a discontinuous gradient medium, wherein the duration of the ultracentrifugation and the relative centrifugal force for the ultracentrifugation are selected such that the viral load is quantitatively transported out of the pancreatin test sample supernatant into a target fraction situated above or in the boundary layer between the overlying, lowest concentration gradient component and the underlying, next higher concentration gradient component, and
e) quantitatively separating the target fraction containing the viral load from the pancreatin test sample supernatant.

In a second embodiment, the invention relates to a process for quantitatively determining the viral load of the pancreatin specimen, in particular the infectious viral load of the pancreatin specimen. In this second embodiment, in addition to the process according to the first embodiment, in a further process step f) after process step e), a quantitative determination of the viral load of the pancreatin specimen is carried out by determining the virus infection titer in the target fraction containing the viral load.

Unless otherwise specified herein, any technical and scientific terms stated below will in each case have the same meaning as they are conventionally understood to have by a person skilled in the particular field of the art. Temperature ranges cited hereinafter in the format of e.g. "0-15°C" denominate a temperature range of from 0°C to 15°C with the range limits being included in each case. Time ranges cited hereinafter in the format of e.g. "30-120 minutes" denominate a time range of from 30 minutes to 120 minutes with the range limits being included in each case. Time ranges cited hereinafter in the format of e.g. "2-8 hours" denominate a time range of from 2 hours to 8 hours with the range limits being included in each case. Ranges of the relative centrifugal force cited hereinafter in the format of e.g. "1,500-5,000 x g" denominate a relative centrifugal force in the range of from 1,500 x g to 5,000 x g with the range limits being included in each case. Volume ranges cited hereinafter in the format of e.g. "10-15 ml" denominate a volume range of from 10 milliliters to 15 milliliters with the range limits being included in each case. Measure of length ranges cited hereinafter in the format of e.g. "80-100 mm" denominate a measure of length range of from 80 millimeters to 100 millimeters with the range limits being included in each case.

The process according to the invention is suitable for all types of pancreatin of animal origin and may in particular be carried out on conventional commercial porcine pancreatins and on bovine pancreatins. The process is preferably carried out on porcine pancreatin specimens.

The process according to the invention is generally suitable for separating the viral load from a pancreatin specimen and for subsequently quantitatively determining the viral load of a pancreatin specimen. In particular, the process is suitable for separating and for quantitatively determining the viral load of pancreatin specimens, in which the viral load comprises bovine rotavirus A, encephalomyocarditis virus (= EMCV), porcine circovirus (= PCV), porcine parvovirus (= PPV), porcine rotavirus A, porcine teschovirus and/or swine vesicular disease virus (= SVDV). Thanks to its very similar properties, human coxsackievirus B 5/1 may be used as a model for verifying the suitability of the process according to the invention for separating and/or quantitatively determining SVDV. Thanks to its very similar properties, bovine rotavirus A (for example strain B 223) may be used as a model for verifying the suitability of the process according to the invention for separating and/or quantitatively determining porcine rotavirus A.

In process step a) of the process according to the invention, a liquid pancreatin test sample suitable for centrifugation is produced from the pancreatin specimen without in so doing changing or modifying the viral load, in particular the infectious viral load, thereof. This may, for example, proceed by producing a pancreatin test sample suspension from the pancreatin specimen. A pancreatin test sample suspension is produced by combining the pancreatin specimen with a cell culture medium which is suitable for the cell line used to culture the virus species to be investigated and with one or more antibiotic(s) suitable for this purpose. Generally, all antibiotics are suitable for producing the antibiotic solution optionally used in process step a). As a rule, broad-spectrum antibiotics or mixtures of such broad-spectrum antibiotics are used. Suitable antibiotics may e.g. be selected from the group comprising β-lactam antibiotics like penicillins, cephalosporins (including oxacephemes and carbacephemes), carbapenemes and monobactames; streptomycin (including streptomycin sulfate); neomycins (including neomycin A, neomycin B and paromomycin); kanamycins (including kanamycin, gentamicin, amicacin and tobramycin); spectinomycin; tetracyclins (including tetracyclin, oxytetracyclin, doxycyclin and minocyclin); macrolide antibiotics (including erythromycin, clarithromycin, roxithromycin, azithromycin, josamycin and spiramycin); gyrase inhibitors (including nalidixin acid, cinoxacin, pipemidic acid, norfloxacin, pefloxacin, ciprofloxacin, ofloxacin and fleroxacin; folic acid antagonists (including sulfonamide antibiotics, diamino benzylpyrimidines and their combinations); chloramphenicol; lincosamides; glycopeptide antibiotics (including vancomycin and teicoplanin); fosfomycin; polypeptide antibiotics (including polymixin B, colistin, bacitracin and tyrothicin) and mupirocin. Preferred antibiotics are streptomycin sulfate and penicillin and mixtures of streptomycin sulfate and penicillin, for example as an antibiotic "cocktail". The one or more antibiotic(s) may for example be used in a solution of a solvent which is suitable for the one or more antibiotic(s) in each case, i.e. as an antibiotic solution.

The pancreatin test sample suspension is conventionally produced with cooling to a temperature of 0-15°C, for example to a temperature of 4-10°C. The constituents for producing the pancreatin test sample suspension are conventionally stirred with ice cooling and for a duration of at least 30 minutes, for example 30-120 minutes, preferably 45-90 minutes, in particular 50 minutes or 60 minutes. The purpose of cooling the pancreatin test sample suspension and of cooling in all further process steps is in each case to avoid or at least substantially reduce any unwanted deactivation of the viral load by the enzymatically active constituents of the pancreatin specimen. In one embodiment, the invention also provides a pancreatin test sample suspension which may be produced according to process step a).

Which cell culture media are in each case used in producing the pancreatin test sample suspension in process step a), is determined by which virus species is to be separated and/or quantitatively determined by the process according to the invention. Permissive cell cultures in which the investigated virus species if possible initiates a cytopathic effect (= CPE) are used for culturing and detecting a specific virus species. CPE is a modification of virus-infected cells which is recognizable by light microscopy. If a virus species multiplies in the culture cell without CPE, such multiplication may in general nevertheless be identified by per se known indirect detection methods.

If, for example, bovine rotavirus A is to be separated and/or quantitatively determined, fetal monkey kidney cells (= MA-104 cells) may, for example, be used for the culturing thereof. In this case, per se known Dulbecco's Modified Eagle Medium (= Dulbecco medium) is, for example, suitable as the cell culture medium. If EMCV is to be separated and/or quantitatively determined, porcine kidney cells (= PK-15 cells) or embryonal porcine kidney cells (= SPEV cells) may be used for the culturing thereof. In the case of PK-15 cells, per se known Minimal Essential Medium (= MEM) is, for example suitable as the cell culture medium. In the case of SPEV cells, Dulbecco medium is, for example, suitable as the cell culture medium. If, for example, PCV is to be separated and/or quantitatively determined, PK-15 cells may, for example, be used for the culturing thereof. If, for example, PPV is to be separated and/or quantitatively determined, porcine kidney cells (SK-6 cells) may, for example, be used for the culturing thereof. In this case, Dulbecco medium is, for example, suitable as the cell culture medium. If, for example, porcine rotavirus A is to be separated and/or quantitatively determined, MA-104 cells may, for example, be used for the culturing thereof. If, for example, porcine teschovirus is to be separated and/or quantitatively determined, PK-15 cells may, for example, be used for the culturing thereof. If, for example, SVDV is to be separated and/or quantitatively determined, SPEV cells may, for example, be used for the culturing thereof. The person skilled in the art is aware of suitable cell lines for culturing the particular virus species and of corresponding suitable cell culture media. Virus species usable according to the present invention and corresponding cell lines may be obtained from appropriate sources, for example from the "American Type Culture Collection", Manassas, USA (= ATCC), the "Deutsche Sammlung von Mikroorganismen und Zellkulturen GmbH", Braunschweig, Germany (= DSMZ), the "Friedrich-Loffler-Institut", Federal Research Institute for Animal Health, Insel Riems, Germany (= FLI) or the "Veterinary Service Division" of the "Department of Agriculture and Rural Development", Belfast, United Kingdom (= DARD).

In process step b), the pancreatin test sample obtained in process step a) may either be used in its entirety, or a defined part of the pancreatin test sample, in particular a defined volume of this pancreatin test sample, may be used. The pancreatin test sample obtained in process step a) is preferably used in its entirety.

In process step b), in the case of low-speed centrifugations, conditions may be established under which viruses with sedimentation constants of ≥ 120 S, in particular of ≥ 120 S to 5,000 S, do not yet form a pellet. Usually viruses with sedimentation constants of ≥ 120 S, in particular viruses with sedimentation constants of ≥ 120 S to 5,000 S, do not yet form pellets when the low-speed centrifugations are carried out with a relative centrifugal force of in each case less than 10,000 × g, preferably of in each case 1,500-5,000 × g, particularly preferably of in each case 2,000-3,500 × g, for example of in each case 2,700 × g. The duration of a low-speed centrifugation step conventionally amounts to at least 5 minutes, usually 5-60 minutes, in particular 10-45 minutes, preferably 10-30 minutes, for example 15 minutes. In a preferred embodiment of process step b), low-speed centrifugation steps are carried out with cooling to a temperature of 0-15°C, for example to a temperature of 4-10°C, preferably in a refrigerated centrifuge.

The purpose of low-speed centrifugation steps in process step b) is primarily to remove from the pancreatin suspension those pancreatin constituents such as insoluble particles etc. which are disruptive in the separation and/or quantitative determination of the viral load of a pancreatin specimen in order to obtain a pancreatin test sample supernatant which is suitable for further processing. The solid deposits optionally obtained in low-speed centrifugation steps are thus generally discarded in process step c), while the supernatant is used for the next process step d). Low-speed centrifugation steps with subsequent discarding of optionally obtained solid deposits are conventionally repeated until solid deposits are no longer observed to form. Usually, no further repetitions will be necessary after 1-3 repetitions, in particular after just one repetition, of low-speed centrifugation steps with subsequent discarding of optionally obtained solid deposits. In an embodiment of the invention, a solid deposit as obtained in process step b) can be a sediment.

pancreatin test sample supernatant obtained after low-speed centrifugation itself, which may then be used in process step d). If a washing fluid other than the pancreatin test sample supernatant itself is used, said washing fluid is combined, once washing of the deposit is complete, with the pancreatin test sample supernatant and then used in process step d). It is particularly advantageous to wash the deposit in the above-stated manner before use in process step d), if the viral load of the pancreatin specimen comprises EMCV.

A discontinuous two-phase sucrose gradient is conventionally used as the discontinuous gradient medium in process step d). The discontinuous gradient medium preferably comprises a gradient prepared from a 50% (wt./vol.) buffered sucrose solution and a 20% (wt./vol.) buffered sucrose solution. Neutral buffers (i.e. which buffer around a pH value of 7) may, for example, be used as the buffer for the sucrose solutions. A PBS buffer (= "phosphate-buffered saline" buffer, pH 7.2) is preferably used. Sterile gradient media are usually used. A two-phase discontinuous sucrose gradient of the above-stated kind provides particularly suitable sedimentation and thus separation conditions for the viruses which may be found. Discontinuous sucrose gradients, in particular a gradient as described above prepared from a 50% (wt./vol.) optionally buffered sucrose solution and a 20% (wt./vol.) buffered sucrose solution, furthermore exhibit suitable osmotic conditions in order not to deactivate any optionally present viral load. Ultracentrifugation according to process step d) ensures, for example, that the viral load originating from the pancreatin specimen is substantially quantitatively transferred from the pancreatin test sample supernatant into the discontinuous gradient medium. Substantially quantitative here means that an viral load previously added to a test sample is so completely recovered that the difference of titer in the added viral load and in that recovered after ultracentrifugation has been carried out is less than or equal to one half step of the base-ten logarithm of virus titer (= 0.5 log steps). The ultracentrifugation according to process step d) furthermore ensures that the viral load is transported into a target fraction which is sufficiently far away from the pancreatin test sample to permit mechanical separation thereof from the pancreatin test sample without it being possible for any remixing of the phases disruptive to the separation to occur.

Process step d) is conventionally carried out by introducing a volume of the highest concentration gradient medium into an ultracentrifuge tube over which is layered a volume of the next lower concentration gradient media. This process is repeated as many

Process step d) is conventionally carried out by introducing a volume of the highest concentration gradient medium into an ultracentrifuge tube over which is layered a volume of the next lower concentration gradient media. This process is repeated as many times as desired to obtain a multi-phase gradient medium with the final (top) layer being a volume of liquid pancreatin test sample suitable for centrifugation from which the viral load is to be separated. In the case of a two-phase gradient medium, the volume of the next lower concentration gradient medium is then immediately overlayed with a volume of a liquid pancreatin test sample or a pancreatin test sample suspension (pancreatin test sample volume) which is suitable for centrifugation. In the case of a two-phase gradient medium, this yields a sequence of phases in the ultracentrifuge tube from the top down of a first layer comprising the pancreatin test sample volume (top), then the volume of the lowest concentration gradient medium (middle; for example a 20% (wt./vol.) buffered sucrose solution) and finally the volume of the highest concentration gradient medium (bottom, covering the base of the ultracentrifuge tube; for example a 50% (wt./vol.) buffered sucrose solution). When overlaying the individual volumes, care must be taken to ensure that no turbulence or intermixing occurs at the respective boundaries.

The target fraction in process step d) typically comprises (i) a part of the lowest concentration gradient medium sufficiently far away and remote from the pancreatin test sample volume and (ii) the complete volume of the next higher concentration gradient medium. In the case of a two-phase gradient medium the target fraction comprises, for example, a part of the lowest concentration gradient medium sufficiently far away and remote from the pancreatin test sample volume and the complete volume of the highest concentration gradient medium extending downward to the bottom of the ultracentrifuge tube.

When calculating the location of a target fraction which is sufficiently removed from the pancreatin test sample volume thereby allowing for subsequent separation, it should be borne in mind that the calculated values for the position of the particles (i.e. the position of the viral particles separated from the pancreatin sample) usually denote the vertices of a Gaussian distribution. As such, the particles will be distributed both above and below their calculated position. It is thus necessary when determining the desired distance of the target fraction from the pancreatin test sample volume to include an additional margin to account for the Gaussian distribution of the particle locations. The viral load is conventionally transported into a target fraction which is sufficiently distant from the pancreatin test sample volume for subsequent separation if particles with a sedimentation constant of ≥ 120 S, in particular of ≥ 120 S to 5,000 S, have migrated from the pancreatin test sample volume at least 10 mm, for example at least 15 mm, at least 20 mm, at least 25 mm or at least 30 mm, into the lowest concentration gradient medium due to the ultracentrifugation. In the case of a two-phase gradient medium previously described, the lowest concentration gradient medium is the 20% (wt./vol.) buffered sucrose solution. In one variant of the ultracentrifugation step, substantially all particles with a sedimentation constant of ≥ 120 S, in particular of ≥ 120 S to 5,000 S, have completely passed through the lowest concentration gradient medium and are concentrated at the boundary layer to the next higher concentration gradient medium (i.e. on a "sucrose cushion"). The person skilled in the art is aware of suitable ways of calculating and implementing the corresponding conditions for ultracentrifugation. Suitable ultracentrifugation conditions may be determined based upon the characteristics of the virus(es) to be separated from the pancreatin sample (e.g., density and sedimentation constant), where applicable assuming per se known simplifications (see for example Lebowitz et al., "Modern analytical ultracentrifugation in protein science: A tutorial review"; Protein Science 11 (2002) 2067-2079).

Provided that ultracentrifugations are carried out using conventional volume ratios and in the discontinuous gradient medium, preferably in the two-phase discontinuous sucrose gradient, the viral load is conventionally transported into a target fraction suitable for subsequent separation, if ultracentrifugation is carried out for a duration of at least 1 hour, usually of at least 2 hours, for example for a duration of 2-8 hours, in particular for a duration of 3-6 hours. A suitable relative centrifugal force for the ultracentrifugation according to the invention is at least 100,000 × g, for example 200,000-350,000 × g. In one embodiment of the ultracentrifugation step, said step is carried out for a duration of 3-6 hours with a relative centrifugal force of 200,000-350,000 × g in volume ratios conventional for carrying out ultracentrifugation and in a gradient prepared from a 50% (wt./vol.) buffered sucrose solution and a 20% (wt./vol.) buffered sucrose solution. In another embodiment of the ultracentrifugation step, said step is carried out for a duration of 3.5-4.5 hours with a relative centrifugal force of 250,000-300,000 × g in volume ratios conventional for carrying out ultracentrifugation and in a gradient prepared from a 50% (wt./vol.) buffered sucrose solution and a 20% (wt./vol.) buffered sucrose solution. Volume ratios conventional for carrying out ultracentrifugation are obtained, for example, if conventional ultracentrifuge tubes are used. Conventional ultracentrifuge tubes are here, for example, taken to be those with a volume of 10-15 ml, in particular 12-13 ml; an internal radius of 6-8 mm, in particular of 7 mm; and a height of 80-100 mm, in particular of 85-95 mm. Provided that a conventional ultracentrifuge tube is used in process step d), the volume of the highest

In a preferred variant of the embodiments of process step d), irrespective of the otherwise selected conditions, ultracentrifugation is carried out with cooling to a temperature of 0-15°C, preferably to a temperature of 4-10°C.

Conventional refrigerated centrifuges which may be used in this process step are known to the person skilled in the art. A conventional commercial ultracentrifuge is conventionally used in process step d), such as a coolable ultracentrifuge with a swinging bucket rotor, for example an ultracentrifuge from Sorvall^{®} with a model "TH-641" swinging-bucket rotor.

The above-stated description of the low-speed centrifugation steps and ultracentrifugation steps according to the invention may be scaled up or down to any desired extent by the person skilled in the art, in particular with the assistance of the further technical information stated in the description of the present invention.

In process step e), the target fraction containing the viral load is quantitatively separated from the pancreatin test sample supernatant. Separation usually proceeds by placing a mark on the ultracentrifuge tube at the height of the previously determined boundary of the target fraction. The entire volume above this boundary is then separated from the remaining volume, for example by being aspirated. Aspiration may, for example, proceed with a conventional peristaltic pump, the tubing and capillaries of which have conveniently previously been sterilized. A suitable pumping rate is, for instance, a rate of 2 ml/minute. During aspiration, care should be taken to ensure that the peristaltic pump capillary is always located at the upper border of the liquid. The target fraction remaining in the ultracentrifuge tube may then be removed from the ultracentrifuge tube in per se known manner with a conventional single channel pipette, preferably with a sterile tip. Any sediment possibly still remaining in the ultracentrifuge tube may be removed at the same time, for example by being repeatedly drawn up and resuspended with the single channel pipette.

If a process for quantitatively determining the viral load of the pancreatin specimen is to be carried out, a process step f) follows process step e). In process step f), the viral load of the pancreatin specimen is quantitatively determined by determining the virus infection titer in the target fraction containing the viral load. Quantitative determination of the virus infection titer in the target fraction may here proceed in accordance with working processes known per se in virology, for example in accordance with the per se known principle of virus infection titer determination (= VITD).

If a process for quantitatively determining the viral load of the pancreatin specimen is to be carried out, a process step f) follows process step e). In process step f), the viral load of the pancreatin specimen is quantitatively determined by determining the virus infection titer in the target fraction containing the viral load. Quantitative determination of the virus infection titer in the target fraction may here proceed in accordance with working processes known per se in virology, for example in accordance with the per se known principle of virus infection titer determination (= VITD).

The target fraction may, for example, be diluted in a suitable ratio with a suitable cell culture medium in order to obtain a virus determination test sample. Suitable cell culture media are those stated above to be usable, in each case as a function of the investigated virus species. In an embodiment of the invention to rule out false positive hits for virus infection, the diluted or undiluted target fraction may be filtered through a microfilter before the quantitative determination of the viral load is carried out in process step f).

A suitable dilution may, for example, be achieved by diluting the target fraction with the cell culture medium to the original volume of the pancreatin test sample supernatant used in process step d). Then, in a first step, a dilution series of the virus determination test samples may first be produced in per se known manner, for example in dilution steps of 1:2, 1:5 or 1:10 or also in combinations of these dilution steps, in order to carry out a quantitative VITD. Then, in a second step, a suitable cell suspension may be inoculated in per se known manner with the virus determination test samples of different concentrations from the dilution series, whereupon a cell layer is allowed to form on the virus determination test samples of different concentrations. To rule out false positive hits for virus infection which may be caused by the presence of microbials such as inert bacteria or mycoplasms, it is then usually expedient to filter the virus determination test samples before inoculating them onto detector cells. To this end, a virus determination test sample or a diluted virus determination test sample may be filtered through a filter of appropriate pore size, such as a microfilter, e.g. a microfilter of a pore size of from 0.1 to 10 µm (range limits included; = microfiltration), usually a microfilter of a pore size of 1 µm. The filtrate may then be used as a test sample for further investigations. Then, in a third step; the inoculated test samples are read for their degree of infection depending on the manner in which their infection is indicated. Where, for example, CPE may be used as an indicator of infection of a cell layer, CPE is read in per se known manner after approximately 4-7 days. Titration (end-point dilution) of the virus determination test samples here permits a quantitative determination of the originally present infection dose. Titration conventionally proceeds by dilution by a factor of 10, i.e. based on the base-ten logarithm. In practice, the 50% infection dose (= ID_{5O}) is usually calculated. In the case of parallel multiple batches, the identified ID₅₀ value then corresponds to that of the highest (reciprocal) dilution of the virus determination test sample at which a CPE is detectable in exactly half the batches. The results may optionally additionally be computationally corrected or interpolated in per se known manner. The most commonly used methods for virus titer calculation are those according to Spearman and Kärber (see C. Spearman, Br. J. Psychol. 2 (1908) 227-242 and G. Kärber, Arch. Exp. Path. Pharmak. 162 (1931) 480-483; also Bundesanzeiger [Federal gazette] no. 84, May 4 1994) or according to Reed and Muench (see Reed, L.J., Muench, H. Am. J. Hyg. 27 (1938) 493-497).

Other indicators of an infection of the cell layer may also be used, for example virus antigen induction or plaque induction. The person skilled in the art is familiar with these methods and their applications in the present case, for example from textbooks of virology such as "Medizinische Virologie" by H.W. Doerr and W.H. Gerlich, Georg Thieme Verlag Stuttgart, New York, 1 st edition 2002 or in each case the most recent edition thereof.

### EXAMPLES

All tasks stated in the following Examples were carried out under sterile conditions on a sterile workbench. The procedures conventional in virological laboratories, for example safety procedures must be observed. The following materials were inter alia used:
1. Antibiotic solution, 1.0 g of streptomycin sulfate and 1.2 g of penicillin are dissolved in 20 ml of twice-distilled water and filtered through a 0.2 µm filter. The filtrates are then divided into 1 ml aliquots and optionally stored at -20°C until use;
2. Dulbecco medium, cell culture medium for SK 6 cells, SPEV cells and MA 104 cells;
3. Single channel pipette, with sterile tips;
4. FCS, fetal calf serum from Bio Whittaker (= serum).
5. Tissue culture flasks, sterile, area of flask base in each case 25, 75 or 175 cm²;
6. MEM, cell culture medium for PK-15 cells with 1.5 g/l sodium bicarbonate and 1 mM pyruvate;
7. Microtiter plates, sterile with 96 wells and lid;
8. PAN suspension,10% pancreatin suspension; 1.0 g of porcine pancreatin (unless otherwise stated) weighed out under sterile conditions into a beaker, combined with 1 ml of antibiotic solution and (unless otherwise stated) 8.0 ml of the particular corresponding cell culture medium and (unless otherwise stated) suspended within 60 minutes in an ice bath with stirring;
9. Pardee buffer, Pardee's carbon dioxide buffer;
10. PBS, sterile "phosphate buffered saline" solution (pH 7.2);
11. Pipettes, sterile;
12. Pipette tips, sterile in sterile trays;
13. Plastics pouches, CO₂-impermeable with closure ("Anaerocult^{®}", from Merck);
14. Polyclonal anti-PPV antibody, fluorescein isothiocyanate (= FITC) conjugate, from NatuTec GmbH;
15. Tubes, sterile 15 and 50 ml;
16. Sucrose solution, 20%, PBS-buffered, sterile; concentration is adjusted in per se known manner with the assistance of a conventional refractometer;
17. Sucrose solutions, 50%, PBS-buffered, sterile; concentration is adjusted in per se known manner with the assistance of a conventional refractometer;
18. Screw-top tubes, sterile;
19. Trypsin solution, "TrypL Express^{®}", from INVITROGEN;
20. Peristaltic pump, from "ismaTec", pumping rate up to 5.8 ml/minute;
21. Refrigerated ultracentrifuge, "Sorvall^{®} Pro 80" with "TH-641" rotor;
22. Ultracentrifuge tubes, sterile, capacity 11 ml, dimensions 9 × 90 mm
23. Dilution blocks, 96 wells each of 1.0 ml;
24. MA-104 cells: supplied by FLI;
25. PK-15 cells: supplied by DARD;
26. SK-6 cells: supplied by FLI;
27. SPEV cells: supplied by FLI;
28. Cell suspensions of the SK 6, SPEV and PK-15 cells to be tested with 200,000 cells/ml in cell culture medium with 10% FCS;
29. Sterile Falcon microtubes, capacity 15 ml

### Example 1: Investigation of the harmful effect of pancreatin on various cell lines

For the purposes of detecting viruses in material test samples using cell cultures, the harmful effect of the pancreatin specimen to be investigated on the cells should be ascertained in order to be able to rule out false negative results when evaluating CPEs. As stated below, investigations to ascertain the harmful effect of a pancreatin test sample suspension were accordingly carried out on various cell lines.

0.5 ml portions of a PAN suspension produced as above were taken for testing the harmful effect and designated "pancreatin suspension test sample".

Low-speed centrifugation: The remaining PAN suspension was centrifuged for 15 minutes at 4,000 rpm (2,700 × g) and 4°C in a conventional refrigerated centrifuge (Megafuge^{®} 1.0R Heraeus SEPATECH^{®} with swinging-bucket rotor no. 2704). The supernatant after low-speed centrifugation was then centrifuged for a further 15 minutes at 4,000 rpm and 4°C and, designated "supernatant after low-speed centrifugation", used for virus titration and ultracentrifugation. The two sediments obtained in each case after the low-speed centrifugations were combined (together 1 ml), resuspended in 9 ml of the respectively suitable cell culture medium and designated "sediment".

Ultracentrifugation: 5.0 ml were taken from the test sample "supernatant after low-speed centrifugation" and subjected to ultracentrifugation in an ultracentrifuge. To this end, 0.5 ml of 50% sucrose solution was introduced by means of a pipette into the number of ultracentrifuge tubes necessary for carrying out testing. With the ultracentrifuge tube held at an oblique angle, this layer was carefully overlayed with 4.5 ml of 20% sucrose solution, a dividing layer being discernible between the two solutions. A 5.0 ml layer of the "supernatant after low-speed centrifugation" test sample taken as stated above was then placed, again with care and avoiding turbulence and intermixing, onto the 20% sucrose solution. The ultracentrifuge tubes were then suspended in the ultracentrifuge rotor. To this end, the two ultracentrifuge tubes on the opposite sides of the rotor were in each case counterbalanced with PBS, inserted in the corresponding holders and tightly sealed with the associated lid. Once the holders had been inserted in the rotor, the test samples were centrifuged for 4 hours at 10°C and 40,000 rpm (273,799 × g). After ultracentrifugation, the ultracentrifuge tubes were removed from the holders on the sterile workbench and provided with a mark at a height of 1.5 cm, measured from the bottom of the ultracentrifuge tube. Using a peristaltic pump, the tubing and capillaries of which had previously been sterilized, the liquid above the mark was aspirated from the ultracentrifuge tube at a pumping rate of 2 ml/minute, the capillary always being located at the upper border of the liquid. This first fraction obtained in this manner was designated "upper fraction after ultracentrifugation". The "lower fraction after ultracentrifugation" (1.5 ml) remaining in the ultracentrifuge tube was in each case removed from the ultracentrifuge tube with the assistance of a single channel pipette. Any sediment possibly remaining on the bottom of the ultracentrifuge tube was resuspended by being repeatedly drawn up with the single channel pipette and likewise removed. The "lower fraction after ultracentrifugation" was made up to 5.0 ml, corresponding to the originally used virus-containing test sample volume, in a sterile graduated tube with a respectively suitable cell culture medium. Until further processing, the two resultant fractions were stored at 4°C or, in the case of extended storage, at - 20°C.

The test samples "pancreatin suspension test sample", "supernatant after low-speed centrifugation", "sediment" (after low-speed centrifugation), "upper fraction after ultracentrifugation" and "lower fraction after ultracentrifugation" (after being made up to 5.0 ml) were then tested for their harmful effect with regard to various cell lines. To this end, dilution series of the test samples to be tested were in each case produced. All the test samples to be tested were further diluted by a factor of 2 from a dilution of 1:5 with the respectively suitable cell culture medium. In microtiter plates, there were added to 100 µl portions of cell suspension comprising PK-15, SPEV or SK 6 cells per well in 8 parallels, 100 µl of the test sample dilutions produced to in each case 100 µl of freshly produced cell suspension. When MA 104 cells were tested, microtiter plates with a 24 hour old cell layer were used. To this end, the cell culture medium was in each case removed from the wells and replaced with 100 µl of fresh cell culture medium without serum, so giving rise to final test sample dilutions of 1:10, 1:20, 1:40, 1:80, 1:160 etc.. As a control, 100 µl of cell culture medium were introduced into eight wells of each microtiter plate instead of 100 µl of the dilution series. Pairs of plates together with a tube containing 4 ml of Pardee buffer and filter paper, were placed in air-tight pouches and tightly sealed with a sealing clip. The plates were then incubated at 36 ± 1°C for up to 7 days. Over the period of incubation, the plates were inspected daily by microscope for the extent of CPE, i.e. for cell lysis and/or degeneration of the cells and the absence of formation of a cell layer as a result of the harmful effect of the pancreatin. The final evaluation was carried out after seven days. The titration was repeated if cell degeneration had already occurred in the controls on the final reading.

Table 1 below shows the results of testing the different test samples for their harmful effect with regard to various cell lines. If a test sample was harmful down to the final dilution of for example 1:640, but no longer harmful at a dilution of 1:1280, then the result stated for this sample in the Table is "test sample harmful ≥ 1:640, but < 1:1280".

**Table 1: Test results of pancreatin suspensions and the subfractions thereof for harmfulness towards various cell lines**

| **Cell lines** | **PK-15** | **MA-104** | **SK 6** | **SPEV** |
|---|---|---|---|---|
| **Test samples** | **Test samples harmful down to a dilution of:** | | | |
| Pancreatin suspension test sample | ≥ 1:640 | ≥ 1:160 | ≥ 1:320 | ≥ 1:320 |
| | < 1:1280 | < 1:320 | < 1:640 | < 1:640 |
| Supernatant after low-speed centrifugation | ≥ 1:640 | ≥ 1:160 | ≥ 1:320 | ≥ 1:320 |
| | < 1:1280 | < 1:320 | < 1:640 | < 1:640 |
| Sediment | ≥ 1:160 | ≥ 1:80 | ≥ 1:80 | ≥ 1:40 |
| | < 1:320 | < 1:160 | < 1:160 | < 1:80 |
| Upper fraction after ultracentrifugation | ≥ 1:320 | ≥ 1:160 | ≥ 1:320 | ≥ 1:320 |
| | < 1:640 | < 1:320 | < 1:640 | < 1:640 |
| Lower fraction after ultracentrifugation | ≥ 1:40 | ≥ 1:20 | ≥ 1:20 | ≥ 1:20 |
| | < 1:80 | < 1:40 | < 1:40 | < 1:40 |

It is clear from the results shown in Table 1 that, in the "lower fraction after ultracentrifugation", which has been subjected to an ultracentrifugation step according to the invention and in which the viral load has been concentrated, there is a considerable reduction in the harmful effect towards the investigated cell lines in comparison with all the other test samples investigated.

If the harmful effect of the untreated pancreatin suspension test sample is compared with that of the lower fractions after ultracentrifugation, it has been possible in the above-described test for MA-104 cells to reduce the harmful effect by a factor of 8, and by a factor of 16 in each case for the three further tested cells. The supernatant was still slightly turbid after the pancreatin suspension test sample had been subjected twice to low-speed centrifugation. During ultracentrifugation, these insoluble particles settle as a thin deposit on the bottom of the ultracentrifuge tube. This deposit was also resuspended and was a constituent of the "lower fraction after ultracentrifugation". It may thus be assumed that this deposit contributes to the residual harmful effect of the "lower fraction after ultracentrifugation" and that its residual harmful effect may be still further reduced by separating and no longer resuspending the above-stated deposit.

### Example 2: Investigation of pancreatin specimens with addition of an elevated virus titer

The aim of the investigations of pancreatin specimens with addition of an elevated virus titer (= "high-spike tests") was inter alia to show that the process according to the invention is suitable for quantitatively separating the viral load from the pancreatin specimen and its constituents which may be harmful to living cells. To this end, high-titer "spiked virus preparations" of each of the viruses to be investigated were prepared in known manner. "High-titer" should here in particular (as a function of the virus species used) in each case be taken to mean a titer of the spiked virus preparation of at least 4 steps of the base-ten logarithm (= log) of the semimaximal "Tissue Culture Infectious Dose" per ml of the investigated test sample (= TCID₅₀/ml). High-titer spiked virus preparations of PCV may, for example, be obtained in accordance with the method of I. Tischer et al., Arch. Virol. 96 (1987) 39-57, by pretreating the PK-15 cells used for culturing with D-(+)-glucosamine solution.

### a. High-spike test with EMCV (strain LC 75)

0.75 ml of a high-titer (7.70 ± 0.10 log TCID₅₀/ml) spiked virus preparation of EMCV (strain LC 75) and 0.75 ml of antibiotic solution were added to a PAN suspension (produced by addition of 4.5 ml of Dulbecco medium to 0.75 g of pancreatin and 50 minutes' stirring with ice cooling) and the resultant suspension was stirred for a further 10 minutes. 0.5 ml of this suspension were taken for virus titration and stored at 4°C until titration (= "EMCV high fraction 1"). The remaining suspension was centrifuged for 15 minutes at 4,000 rpm (= 2,700 × g) and 4°C. The supernatant after centrifugation was recentrifuged in a new centrifuge tube for 15 minutes at 4°C and 4,000 rpm. The supernatant after the second centrifugation was quantitatively transferred into a sterile tube (= "EMCV high fraction 2"). The two sediments after low-speed centrifugation were resuspended with a total of 6.5 ml of Dulbecco medium, combined and recentrifuged for 15 minutes at 4,000 rpm and 4°C. The resultant supernatant was transferred into a sterile tube. Washing of the sediment was repeated twice more, each time with a 6.5 ml portion of fresh Dulbecco medium. The three washing solutions were combined and used for the virus titration (= "EMCV high fraction 3"). After three washings, the sediment was resuspended in 6.5 ml of Dulbecco medium and then titrated (= "EMCV high fraction 4").

5.0 ml of EMCV high fraction 2 were subjected to an ultracentrifugation in the discontinuous sucrose gradient as described above in Example 1. After ultracentrifugation, the upper (= "EMCV high fraction 5") and lower (= "EMCV high fraction 6") fractions were obtained separately and titrated.

Virus dilution series by a factor of 3 were produced and transferred each with 12 dilution steps in 12 parallels onto microtiter plates with SPEV cell suspension. Spike virus - titration from dilution 10⁻³; EMCV high fraction 1 - titration from dilution 10⁻²; EMCV high fraction 2 - titration from dilution 10⁻²; EMCV high fraction 4 - titration from dilution 10⁻¹; EMCV high fraction 3 - titration from dilution 10⁻²; EMCV high fraction 5 - titration from undiluted test sample; EMCV high fraction 6 - triplicate titration from dilution 10⁻³. The microtiter plates are incubated at 36 ± 1°C in an approx. 5% CO₂ atmosphere and, over the course of 6-7 days, evaluated by microscope for the development of CPE in the wells. Titer is calculated in the test samples in accordance with the Spearman-Kärber method. The results of the high-spike test with EMCV are shown in Table 2 below.

**Table 2: Results of the high-spike tests with EMCV in pancreatin suspensions**

| **Test sample** | **Titer log TCID₅₀/ml**¹⁾ | **Test sample volume [ml]** | **Virus load [log TCID₅₀/ml + log volume]**²⁾ |
|---|---|---|---|
| Spike virus (EMCV) | 7.70 ± 0.10 | 0.75 | 7.58 ± 0.20 |
| EMCV high fraction 1 | 7.14 ± 0.10 | 7.5 | 8.02 ± 0.20 |
| EMCV high fraction 2 | 7.38 ± 0.09 | 5 | 8.08 ± 0.18 |
| EMCV high fraction 4 | 3.32 ± 0.08 | 7.5 | 4.20 ± 0.16 |
| EMCV high fraction 3 | 5.67 ± 0.10 | 19.5 | 6.96 ± 0.20 |
| EMCV high fraction 5 | 4.71 ± 0.10 | 8.5 | 5.64 ± 0.20 |
| EMCV high fraction 6 (1) | 6.99 ± 0.11 | 5 | 7.69 ± 0.22 |
| EMCV high fraction 6 (2) | 7.07 ± 0.10 | 5 | 7.77 ± 0.20 |
| EMCV high fraction 6 (3) | 6.99 ± 0.10 | 5 | 7.69 ± 0.20 |
| Average of the 3 fractions for EMCV high fraction 6³⁾ | 7.02 ± 0.05 ³⁾ | 5 | 7.72 ± 0.10 |

| | | | |
|---|---|---|---|
| ¹⁾ Value is the standard deviation of the individual titration; ²⁾ Value is the 95% confidence interval; ³⁾ Value is the standard deviation of 3 determinations | | | |

It is clear from Table 2 that, when the process according to the invention is carried out, the viral load of the untreated spike test samples (EMCV high fractions 1 and 2), taking account of a generally accepted range of variation of 0.5 log steps, was at least approximately quantitatively recovered in the lower fraction after ultracentrifugation (EMCV high fraction 6). It may furthermore be concluded from the test results that the pancreatin specimen itself had no inhibiting or inactivating action on EMCV.

### b. High-spike test with porcine parvovirus

Porcine parvovirus (PPV) can be cultured in growing SK 6 cells. If the virus yield is too low, the virus is concentrated after cultivation.

1.0 ml of a high-titer (4.75 ± 0.06 log TCID₅₀/ml) spiked virus preparation of PPV were added to a PAN suspension (produced by addition of 7.0 ml of Dulbecco medium and 50 minutes' stirring with ice cooling) and the resultant suspension was stirred for a further 10 minutes. 0.5 ml of this suspension were taken for virus titration and stored at 4°C until titration (= "PPV high fraction 1"). The remaining suspension was centrifuged for 15 minutes at 4,000 rpm (= 2,700 × g) and 4°C. The supernatant after centrifugation was recentrifuged in a new centrifuge tube for 15 minutes at 4°C and 4,000 rpm. The supernatant after the second centrifugation was quantitatively transferred into a sterile tube (= "PPV high fraction 2"). The two sediments after low-speed centrifugation were resuspended with a total of 9 ml of Dulbecco medium, combined and recentrifuged for 15 minutes at 4,000 rpm and 4°C. The resultant supernatant was transferred into a sterile tube. Washing of the sediment was repeated twice more, each time with a 9 ml portion of fresh Dulbecco medium. The three washing solutions were then combined and used for the virus titration (= "PPV high fraction 3"). After three washings, the sediment was resuspended in 9 ml of Dulbecco medium and then titrated (= "PPV high fraction 4").

5.0 ml of PPV high fraction 2 were subjected to an ultracentrifugation in the discontinuous sucrose gradient as described above in Example 1. After ultracentrifugation, the upper (= "PPV high fraction 5") and lower (= "PPV high fraction 6") fractions were obtained separately and titrated.

Virus dilution series by a factor of 3 were produced and transferred each with 12 dilution steps in 12 parallels onto microtiter plates with SK 6 cell suspension. The microtiter plates were incubated at 36 ± 1 °C in an approx. 5% CO₂ atmosphere and, over the course of 6-7 days, evaluated by microscope for the development of CPE in the wells. After this incubation period, the plates were fixed by addition of an ice-cold acetone/methanol mixture and the wells with uncertain CPE were incubated for the final titer determination additionally with FITC-labeled anti-PPV antibody and then evaluated under a UV light microscope. Titer is calculated in the test samples in accordance with the Spearman-Kärber method.

**Table 3: Results of the high-spike tests with PPV in pancreatin suspensions**

| **Test sample** | **Titer log TCID₅₀/ml¹⁾** | **Test sample volume [ml]** | **Virus load [log TCID₅₀/ml + log volume]**²⁾ |
|---|---|---|---|
| Spike virus (PPV) | 4. 95 ± 0. 15 | 1 | 4.95 ± 0.30 |
| PPV high fraction 1 | 3.56 ± 0.08 | 10 | 4.56 ± 0.16 |
| PPV high fraction 2 | 4.47 ± 0.08 | 5 | 5.17 ± 0.16 |
| PPV high fraction 4 | 2.08 ± 0.11 | 10 | 3.08 ± 0.22 |
| PPV high fraction 3 | 2.38 ± 0.09 | 27 | 3.81 ± 0.18 |
| PPV high fraction 5 | < 3.15 | 8.5 | < 4.08 |
| PPV high fraction 6 (1) | 4.67 ± 0 | 5 | 5.37 ± 0 |
| PPV high fraction 6 (2) | 4.43 ± 0.08 | 5 | 5.13 ± 0.16 |
| PPV high fraction 6 (3) | 4.47 ± 0.08 | 5 | 5.17 ± 0.16 |
| Average of the 3 fractions for PPV high fraction 6³⁾ | 4.52 ± 0.13³⁾ | 5 | 5.22 ± 0.26 |

| | | | |
|---|---|---|---|
| ¹⁾ Value is the standard deviation of the individual titration; ²⁾ Value is the 95% confidence interval; ³⁾ Value is the standard deviation of 3 determinations | | | |

It is clear from Table 3 that, as shown above in the EMCV high spike experiment, if the process according to the invention proceeds successfully, the viral load of the untreated spike test samples (PPV high fractions 1 and 2), taking account of a generally accepted range of variation of 0.5 log steps, is at least approximately quantitatively recovered in the lower fraction after ultracentrifugation (PPV high fraction 6). In the PPV high fraction 1, i.e. in the presence of insoluble particles, a one log-step lower titer is determined than in PPV high fraction 2. The presence of insoluble constituents thus apparently disrupts titration. It may furthermore be concluded from the test results that the pancreatin specimen itself had no inhibiting or inactivating action on PPV.

### Example 2: Investigation of pancreatin specimens with addition of a low virus titer

The aim of the investigations of pancreatin specimens with addition of falling virus titers (= "low-spike tests") was inter alia to ascertain the detection limit of the process according to the invention for the virus used in each case. Quantitative detection of the viral load in the individual test samples with a falling virus titer is deemed successful if the virus titer of the originally added spiked virus preparation, taking account of a generally accepted range of variation of 0.5 log steps, is at least approximately quantitatively recovered in the lower fractions after ultracentrifugation.

### a. Low-spike test with EMCV

A PAN suspension was produced (with 2.5 g of porcine pancreatin, 2.5 ml of antibiotic solution, 20 ml of Dulbecco medium). The low-spike test was then carried out in duplicate in mutually independent tests:

In the first test, the batches stated below were produced from PAN suspension plus EMCV spike virus solution:
1) 4.5 ml of pancreatin suspension + 0.5 ml of 10⁻³ dilution of spike virus; resultant dilution 10⁻⁴;
2) 4.5 ml of pancreatin suspension + 0.5 ml of 10⁻⁴ dilution of spike virus; resultant dilution 10⁻⁵_{;}
3) 4.5 ml of pancreatin suspension + 0.5 ml of 10⁻⁵ dilution of spike virus; etc.
4) 4.5 ml of pancreatin suspension + 0.5 ml of 10⁻⁶ dilution of spike virus;
5) 4.5 ml of pancreatin suspension + 0.5 ml of 10⁻⁷ dilution of spike virus;
6) 4.5 ml of pancreatin suspension + 0.5 ml of 10⁻⁸ dilution of spike virus.

All the batches were incubated for 1 hour at room temperature and then subjected to low-speed centrifugation for 15 minutes at 4°C and 4,000 rpm (2,700 x g). The supernatants after low-speed centrifugation were in each case transferred into fresh centrifuge tubes and subjected to another low-speed centrifugation under the same conditions. The supernatants after the low-speed centrifugations were if necessary made up to a volume of 5.0 ml with cell culture medium and in each case 5.0 ml of the supernatants after low-speed centrifugations were subjected to ultracentrifugation in the discontinuous sucrose gradient as described above in Example 1. After ultracentrifugation, the upper fractions (= "EMCV low fraction 2"; down to 1.5 cm of the ultracentrifuge tube) were in each case pumped out with a peristaltic pump and the respective lower fractions (= "EMCV low fraction 3") were removed from the ultracentrifuge tube with a pipette. The lower fractions after ultracentrifugation were in each case made up to 5.0 ml with MEM and then used for the titrations or culturing in tissue culture flasks.

Virus dilution series by a factor of 3 were then produced from each of the above-stated batches 1) to 3) and transferred each with 12 dilution steps in 12 parallels onto microtiter plates with SPEV cell suspension (100 µl portion per well of freshly prepared cell suspension of SPEV cells with 200,000 cells/ml). The test samples of the EMCV low fraction 3 of all the batches were additionally transferred into cell culture flasks with a base area of 25 cm² and 10 ml portions of freshly prepared cell suspension of SPEV cells with 200,000 cells/ml. To this end, 6 tissue culture flasks for each test sample were infected with a 0.2 ml portion of the test sample EMCV low fraction 3. In parallel, a control was provided by one tissue culture flask without any addition. All the titration plates and tissue culture flasks were incubated at 36 ± 1 °C and, over the course of 6-7 days, evaluated by microscope for the development of CPE in the wells or tissue culture flasks. Titer is calculated in the titrated test samples in accordance with the Spearman-Kärber method.

If no CPE was observed in any of the 6 tissue culture flasks of a batch after 7 days, these flasks were three times frozen at -70°C and thawed again. The contents of all the tissue culture flasks were combined and filtered through a 0.1 µm (pore size) filter. The resulting filtrate was used to prepare a second pass on SPEV cell suspension: 2 tissue culture flasks each comprising 10 ml of fresh SPEV cell suspension were infected with 2 ml of the suspension obtained from the 1st pass and likewise incubated for up to 7 days at 36 ± 1°C and observed for the development of CPE. If no CPE was observed in the 2nd pass too, a 3rd pass was additionally carried out. If a negative result was also found in the 3rd pass, the original test sample may be deemed to be free of EMCV.

A detection limit of the process according to the invention of one infectious unit of EMCV per 0.1 g of pancreatin specimen used was ascertained in the above-stated low-spike tests with graduated dilutions of EMCV.

### b. Low-spike test with PPV

A PAN suspension was produced (with 2.5 g of porcine pancreatin, 2.5 ml of antibiotic solution, 20 ml of Dulbecco medium). The low-spike test was then carried out in duplicate in mutually independent tests:

To this end, the batches stated below were produced from PAN suspension plus PPV spike virus solution:
1) 4.5 ml of pancreatin suspension + 0.5 ml of 10⁻¹ dilution of spike virus;
2) 4.5 ml of pancreatin suspension + 0.5 ml of 10⁻² dilution of spike virus;
3) 4.5 ml of pancreatin suspension + 0.5 ml of 10⁻³ dilution of spike virus;
4) 4.5 ml of pancreatin suspension + 0.5 ml of 10⁻⁴ dilution of spike virus;

All the batches were incubated for 1 hour at room temperature and then subjected to low-speed centrifugation for 15 minutes at 4°C and 4,000 rpm (2,700 × g). The supernatants after low-speed centrifugation were in each case transferred into fresh centrifuge tubes and subjected to another low-speed centrifugation under the same conditions. The supernatants after the low-speed centrifugations were if necessary made up to a volume of 5.0 ml with cell culture medium and in each case 5.0 ml of the supernatants after low-speed centrifugations were subjected to ultracentrifugation in the discontinuous sucrose gradient as described above in Example 1. After ultracentrifugation, the upper fractions (= "PPV low fraction 2"; down to 1.5 cm of the ultracentrifuge tube) were in each case pumped out with a peristaltic pump and the respective lower fractions (= "PPV low fraction 3") were removed from the ultracentrifuge tube with a pipette. The lower fractions after ultracentrifugation were in each case made up to 5.0 ml with Dulbecco medium and then used for the titrations or culturing in tissue culture flasks.

Virus dilution series by a factor of 3 were then produced from each of the above-stated batches 1) to 3) and transferred each with 12 dilution steps in 8 parallels onto microtiter plates with SK-6 cell suspension (100 µl portion per well of freshly prepared cell suspension of SK-6 cells with 200,000 cells/ml).

6 tissue culture flasks for each of the batches were infected with a 0.2 ml portion of PPV low fraction 3. In parallel, a control was provided by one tissue culture flask without any addition. All the titration plates and tissue culture flasks were incubated at 36 ± 1°C and, over the course of 6-7 days, evaluated by microscope for the development of CPE in the wells or tissue culture flasks. Titer is calculated in the titrated test samples in accordance with the Spearman-Kärber method.

If no CPE was observed in any of the 6 tissue culture flasks of a batch after 7 days, these flasks were three times frozen at -70°C and thawed again. The contents of all the tissue culture flasks were combined and filtered through a 0.1 µm (pore size) filter. The resulting filtrate was used to prepare a second pass on SK-6 cell suspension: 2 tissue culture flasks each comprising 10 ml of fresh SK-6 cell suspension were infected with 2 ml of the suspension obtained from the 1 st pass and likewise incubated for up to 7 days at 36 ± 1°C and observed for the development of CPE. If no CPE was observed in the 2nd pass too, a 3rd pass was additionally carried out. If no CPE was found in the 3rd pass either, the cell culture medium was removed from the 3rd pass flasks after 7 days and the cell layer was fixed with ice-cold acetone/methanol (80:20 vol./vol.). Infected cells were then detected in the tissue culture flasks by means of FITC-labeled anti-PPV antibody (with 1 ml of antibody solution per flask; incubation for 60 minutes at 37°C, washing with washing buffer and subsequent evaluation by microscope under UV light). If the 3rd pass flasks were free of infected cells, the original test sample was deemed to be free of PPV.

A detection limit of the process according to the invention of one infectious unit of PPV per 0.1 g of pancreatin specimen used was ascertained in the above-stated low-spike tests with graduated dilutions of PPV.

## Claims

1. A process for separating a viral load from a pancreatin specimen, comprising the process steps:
a) producing a liquid pancreatin test sample suitable for centrifugation from the pancreatin specimen without in so doing changing the viral load thereof,
b) subjecting at least one defined part of the pancreatin test sample from process step a) to at least one low-speed centrifugation under conditions, under which viruses with sedimentation constants of ≥ 120 S do not yet form a pellet,
c) discarding any solid deposit arising in process step b) during low-speed centrifugation and retaining a pancreatin test sample supernatant,
d) subjecting at least one defined part of the pancreatin test sample supernatant obtained in process step c) to ultracentrifugation in a discontinuous gradient medium for a duration of at least 1 hours, wherein the relative centrifugal force is at least 100,000 x g, and
e) quantitatively separating the target fraction containing the viral load from the pancreatin test sample supernatant.

2. The process as claimed in claim 1, in which additionally in a process step f) after process step e) a quantitative determination of the viral load of the pancreatin specimen is carried out by determining the virus infection titer in the target fraction containing the viral load.

3. The process as claimed in claim 2, in which the diluted or undiluted target fraction is filtered through a microfilter before the quantitative determination of the viral load is carried out.

4. The process as claimed in claim 1, in which in process step a) the pancreatin test sample is produced as a pancreatin test sample suspension by combining the pancreatin specimen with a cell culture medium which is suitable for the cell line used to culture the virus type to be investigated and with one or more antibiotics.

5. The process as claimed in claim 4, in which production of the pancreatin test sample suspension proceeds with cooling to a temperature of 0-15°C.

6. The process as claimed in claim 1, in which low-speed centrifugations in process step b) are in each case carried out with a relative centrifugal force of less than 10,000 × g.

7. The process as claimed in claim 1, in which low-speed centrifugations in process step b) are in each case carried out with a relative centrifugal force of 1,500-5,000 × g.

8. The process as claimed in claim 1, in which low-speed centrifugations in process step b) are carried out for a duration of at least 5 minutes.

9. The process as claimed in claim 8, in which the ultracentrifugation in process step d) is carried out for a duration of 2-8 hours and the relative centrifugal force is 200,000-350,000 × g.

10. The process as claimed in claim 1, in which low-speed centrifugations in process step b) and the ultracentrifugation in process step d) are in each case carried out with cooling to a temperature of 0-15°C.

11. The process as claimed in claim 1, in which the discontinuous gradient medium introduced in process step d) is a discontinuous two-phase sucrose gradient.

12. The process as claimed in claim 11, in which the discontinuous gradient medium is a gradient prepared from a 50% (wt./vol.) buffered sucrose solution and a 20% (wt./vol.) buffered sucrose solution.

13. The process as claimed in claim 1, in which the pancreatin specimen is a porcine pancreatin specimen.

14. The process as claimed in claim 1, in which the viral load of the pancreatin test sample comprises bovine rotavirus A, encephalomyocarditis virus, porcine circovirus, porcine parvovirus, porcine rotavirus A, porcine teschovirus and/or swine vesicular disease virus.

## Patentansprüche

1. Verfahren zur Abtrennung einer Viruslast von einem Pankreatinmuster, umfassend die Verfahrensschritte
a) Herstellen einer zur Zentrifugation geeigneten flüssigen Pankreatintestprobe aus dem Pankreatinmuster, ohne dabei dessen Viruslast zu verändern,
b) Unterwerfen mindestens eines definierten Teiles der Pankreatintestprobe aus Verfahrensschritt a) mindestens einer niedrigtourigen Zentrifugation unter Bedingungen, bei denen Viren mit Sedimentationskonstanten ≥ 120 S noch nicht pelletieren,
c) Verwerfen von in Verfahrensschritt b) bei niedrigtouriger Zentrifugation gegebenenfalls entstandenem Niederschlag und Zurückbehalten eines Pankreatintestproben-Überstandes,
d) Unterwerfen mindestens eines definierten Teiles des in Verfahrensschritt c) erhaltenen Pankreatintestproben-Überstandes einer Ultrazentrifugation in einem diskontinuierlichen Gradientenmedium für die Dauer mindestens einer Stunde, wobei die relative Zentrifugalkraft wenigstens 100.000 x g beträgt, und
e) quantitatives Abtrennen der die Viruslast enthaltenden Zielfraktion von dem Pankreatintestproben-Überstand.

2. Verfahren nach Anspruch 1, bei dem zusätzlich in einem Verfahrensschritt f) nach Verfahrensschritt e) eine quantitative Bestimmung der Viruslast des Pankreatinmusters durchgeführt wird, indem man den Virusinfektionstiter in der die Viruslast enthaltenden Zielfraktion bestimmt.

3. Verfahren nach Anspruch 2, bei dem vor der Durchführung der quantitativen Bestimmung der Viruslast die verdünnte oder unverdünnte Zielfraktion durch ein Mikrofilter filtriert wird.

4. Verfahren nach Anspruch 1, bei dem in Verfahrensschritt a) die Pankreatintestprobe als Pankreatintestprobensuspension hergestellt wird, indem man das Pankreatinmuster mit einem Zellkulturmedium, das für die zur Kultivierung des zu untersuchenden Virustyps verwendete Zelllinie geeignet ist, sowie mit einem oder mehreren Antibiotika versetzt.

5. Verfahren nach Anspruch 4, bei dem die Herstellung der Pankreatintestprobensuspension unter Abkühlen auf eine Temperatur von 0-15°C verläuft.

6. Verfahren nach Anspruch 1, bei dem niedrigtourige Zentrifugationen in Verfahrensschritt b) jeweils bei einer relativen Zentrifugalkraft von weniger als 10.000 x g durchgeführt werden.

7. Verfahren nach Anspruch 1, bei dem niedrigtourige Zentrifugationen in Verfahrensschritt b) jeweils bei einer relativen Zentrifugalkraft von 1.500-5.000 x g durchgeführt werden.

8. Verfahren nach Anspruch 1, bei dem niedrigtourige Zentrifugationen in Verfahrensschritt b) für eine Dauer von wenigstens 5 Minuten durchgeführt werden.

9. Verfahren nach Anspruch 8, bei dem die Ultrazentrifugation in Verfahrensschritt d) für eine Dauer von 2-8 Stunden durchgeführt wird und die relative Zentrifugalkraft 200.000-350.000 x g beträgt.

10. Verfahren nach Anspruch 1, bei dem niedrigtourige Zentrifugationen in Verfahrensschritt b) und die Ultrazentrifugation in Verfahrensschritt d) jeweils unter Abkühlen auf eine Temperatur von 0-15°C durchgeführt werden.

11. Verfahren nach Anspruch 1, bei dem es sich bei dem in Verfahrensschritt d) eingeführten diskontinuierlichen Gradientenmedium um einen diskontinuierlichen zweiphasigen Saccharosegradienten handelt.

12. Verfahren nach Anspruch 11, bei dem es sich bei dem diskontinuierlichen Gradientenmedium um einen aus einer 50%igen (Gew./Vol.) gepufferten Saccharoselösung und einer 20%igen (Gew./Vol.) gepufferten Saccharoselösung hergestellten Gradienten handelt.

13. Verfahren nach Anspruch 1, bei dem es sich bei dem Pankreatinmuster um ein Schweinepankreatinmuster handelt.

14. Verfahren nach Anspruch 1, bei dem die Viruslast der Pankreatintestprobe das Bovine Rotavirus A, das Encephalomyocarditis-Virus, das Porcine Circovirus, das Porcine Parvovirus, das Porcine Rotavirus A, das Porcine Teschovirus und/oder das Swine Vesicular Disease-Virus umfasst.

## Revendications

1. Procédé pour séparer une charge virale d'un spécimen de pancréatine, comprenant les étapes de procédé consistant à :
a) produire un échantillon d'essai de pancréatine liquide adapté pour centrifugation à partir du spécimen de pancréatine sans changer de cette façon la charge virale de celui-ci,
b) soumettre au moins une partie définie de l'échantillon d'essai de pancréatine provenant de l'étape de procédé a) à au moins une centrifugation à basse vitesse dans des conditions dans lesquelles les virus avec des constantes de sédimentation de ≥ 120 S ne forment cependant pas un culot,
c) éliminer tout dépôt solide formé dans l'étape de procédé b) pendant la centrifugation à basse vitesse et conserver un surnageant d'échantillon d'essai de pancréatine,
d) soumettre au moins une partie définie du surnageant d'échantillon d'essai de pancréatine obtenu dans l'étape de procédé c) à une ultracentrifugation dans un milieu à gradient discontinu pendant une durée d'au moins 1 heure, où la force centrifuge relative est d'au moins 100 000 x g, et
e) séparer quantitativement la fraction cible contenant la charge virale du surnageant d'échantillon d'essai de pancréatine.

2. Procédé selon la revendication 1, dans lequel, en outre, dans une étape de procédé f) après l'étape de procédé e), une détermination quantitative de la charge virale du spécimen de pancréatine est conduite par détermination du titre d'infection virale dans la fraction cible contenant la charge virale.

3. Procédé selon la revendication 2, dans lequel la fraction cible diluée ou non diluée est filtrée à travers un microfiltre avant que la détermination quantitative de la charge virale soit conduite.

4. Procédé selon la revendication 1, dans lequel, dans l'étape de procédé a), l'échantillon d'essai de pancréatine est produit sous la forme d'une suspension d'échantillon d'essai de pancréatine par combinaison du spécimen de pancréatine avec un milieu de culture de cellules qui est adapté pour la lignée cellulaire utilisée pour cultiver le type de virus à rechercher et avec un ou plusieurs antibiotiques.

5. Procédé selon la revendication 4, dans lequel la production de la suspension d'échantillon d'essai de pancréatine est conduite avec un refroidissement à une température de 0 à 15 °C.

6. Procédé selon la revendication 1, dans lequel les centrifugations à basse vitesse dans l'étape de procédé b) sont dans chaque cas conduites à une force centrifuge relative de moins de 10 000 x g.

7. Procédé selon la revendication 1, dans lequel les centrifugations à basse vitesse dans l'étape de procédé b) sont dans chaque cas conduites avec une force centrifuge relative de 1 500 à 5 000 x g.

8. Procédé selon la revendication 1, dans lequel les centrifugations à basse vitesse dans l'étape de procédé b) sont conduites pendant une durée d'au moins 5 minutes.

9. Procédé selon la revendication 8, dans lequel l'ultracentrifugation dans l'étape de procédé d) est conduite pendant une durée de 2 à 8 heures et la force centrifuge relative est de 200 000 à 350 000 x g.

10. Procédé selon la revendication 1, dans lequel les centrifugations à basse vitesse dans l'étape de procédé b) et l'ultracentrifugation dans l'étape de procédé d) sont dans chaque cas conduites avec refroidissement à une température de 0 à 15 °C.

11. Procédé selon la revendication 1, dans lequel le milieu à gradient discontinu introduit dans l'étape de procédé d) est un gradient de saccharose à deux phases discontinu.

12. Procédé selon la revendication 11, dans lequel le milieu de gradient discontinu est un gradient préparé à partir d'une solution de saccharose tamponnée à 50 % (poids/vol.) et une solution de saccharose tamponnée à 20 % (poids/vol.).

13. Procédé selon la revendication 1, dans lequel le spécimen de pancréatine est un spécimen de pancréatine porcine.

14. Procédé selon la revendication 1, dans lequel la charge virale de l'échantillon d'essai de pancréatine comprend le rotavirus A bovin, le virus de l'encéphalomyocardite, le circovirus porcin, le parvovirus porcin, le rotavirus A porcin, le teschovirus porcin et/ou le virus de la maladie vésiculeuse du porc.
